(19) Europäisches Patentamt
European Patent Office
Office européen des brevets

(11) Publication number: **0 127 455**
**B1**

# (12) EUROPEAN PATENT SPECIFICATION

(45) Date of publication of patent specification: **06.09.89**

(51) Int. Cl.$^4$: **C 09 B 23/10** // G03C1/22

(21) Application number: **84303540.3**

(22) Date of filing: **25.05.84**

## (54) Merocyanine dyes.

(30) Priority: **27.05.83 GB 8314800**

(43) Date of publication of application:
**05.12.84 Bulletin 84/49**

(45) Publication of the grant of the patent:
**06.09.89 Bulletin 89/36**

(84) Designated Contracting States:
**CH DE FR GB LI**

(56) References cited:
**BE-A- 674 061**
**DE-A-1 804 972**
**FR-A-1 446 107**

(73) Proprietor: **MINNESOTA MINING AND MANUFACTURING COMPANY**
**3M Center**
**Saint Paul, Minnesota 55101 (US)**

(72) Inventor: **Lea, Bernard Alan**
**45 High View Road**
**South Woodford London E18 2HL (GB)**

(74) Representative: **Bowman, Paul Alan et al**
**LLOYD WISE, TREGEAR & CO. Norman House**
**105-109 Strand**
**London WC2R OAE (GB)**

**EP 0 127 455 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a new class of merocyanine dyes which have been found to possess unexpected and advantageous properties for the spectral sensitisation of dry silver systems.

The class of merocyanine dyes has been extensively reported in the literature, e.g. The Theory of the Photographic Process, C. E. K. Mees, T. H. James, 3rd Edition, The MacMillan Company; Hamer-Cyanine dyes and related compounds, Interscience 1964, JACS *73* 5326—8; British Patent Specification Nos. 428 222, 428 359 and 519 895 and United States Patent Specification Nos. 2 493 748 and 3 719 495. Merocyanine dyes have been used extensively as spectral sensitisers for conventional wet processed silver halide photographic materials and to a lesser extent as spectral sensitisers in dry silver systems.

Dry silver photosensitive compositions comprise an intimate mixture of a light sensitive silver halide and another silver compound such as a silver salt of an organic acid, e.g. silver behenate or silver saccharine, which upon reduction gives a visible change and which is substantially light sensitive. Such a mixture is usually prepared in suspension and the resulting dispersion spread as a layer on a suitable substrate. When dry, the layer is exposed to a light image and thereafter a reproduction of the image can be developed by heating the layer in the presence of a reducing agent such as hydroquinone or certained substituted phenols.

It is because the exposure and development of the layer occur without using water, that these materials are often referred to as dry silver light sensitive materials. Such materials, in which minor amounts of a photosensitive silver halide catalyst progenitor are associated in catalytic proximity with major amounts of a heat sensitive oxidation-reduction image forming reaction mixture which reacts more rapidly in the presence of the catalyst resulting upon exposure of the silver halide, are well known in the art. Examples of such materials are described in British Patent Specification No. 1 110 046 and United States Patent Specification No. 3 839 049 and 3 457 075. United States Patent Specification No. 3 719 495 discloses the use of certain tri-nuclear merocyanine dyes as red sensitisers in such dry silver systems.

It has now been found that certain merocyanine dyes possess unexpected and advantageous properties which render the compounds particularly suitable for use in dry silver systems.

Therefore according to the present invention there is provided a compound of the general formula:

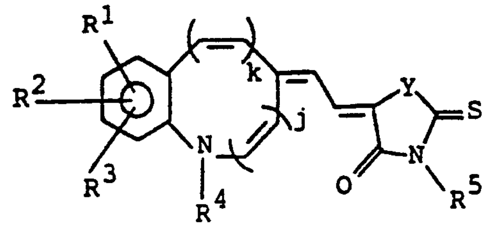

in which:

$R^1$, $R^2$ and $R^3$ independently represent a hydrogen atom or a substituent compatible with cyanine dyes, $k + j = 1$,

$R^4$ represents a lower alkyl group containing 1 to 4 carbon atoms, $(CH_2)_nCOOX$, in which n is an integer from 1 to 10, and X is a cation, e.g. hydrogen or a metal or ammonium ion,

$Y = S$ or $> N—R^6$, and

$R^5$ and $R^6$ independently represent a lower alkyl group of 1 to 4 carbon atoms, cyclohexyl, phenyl, $(CH_2)_nCOOX$ in which n and X are as defined above,

with the proviso that at least one of $R^4$, $R^5$ and $R^6$ represents $(CH_2)_nCOOX$ in which n is at least 4, preferably at least 5.

It has been found that merocyanine dyes of the above formula not only give excellent spectral sensitisation to dry silver systems but also exhibit good stability before coating, good thermal stability when drying after coating and have good latitude with respect to the quantity required. The dyes are advantageous with respect to known merocyanine dyes which have been used to sensitise dry silver systems and have superior properties to merocyanine dyes of similar structure.

It is believed that these unexpected properties may be attributed to the presence of the group $(CH_2)_nCOOX$ in which n is at least 4. It has surprisingly been found that the presence of a long chain (at least 4 carbon atoms) linking the carboxyl group provides improved properties compared to the same dye having a short chain length linking the carboxyl group.

The substituents $R^1$, $R^2$ and $R^3$ may be the same as or different from one another and each represents a substituent which can be present in a cyanine dye type heterocyclic nucleus. Such substituents are known in the art and include hydrogen or halogen, e.g. chlorine, bromine or iodine, an alkyl or alkoxy group containing 1 to 4 carbon atoms, an alkenyl group containing 2 to 4 carbon atoms, alkaryl or aralkyl group of up to 12 carbon atoms, phenyl, $—(CH_2)_mCOOH$ in which m is 0, 1, 2 or 3, $—NO_2$, $—NH_2$, or $—NHCOCH_3$, or any two adjacent groups of $R^1$, $R^2$ and $R^3$ together represent the carbon atoms needed to complete a fused on benzene ring. Preferably at least one, more preferably at least two, of the substituents $R^1$, $R^2$ and $R^3$ represent hydrogen atoms. The most preferred substituents to be represented by each of $R^1$, $R^2$ and $R^3$ are

hydrogen, chlorine or bromine atoms, or methyl, ethyl, methoxy or ethoxy groups.

Preferably $R^4$ is $C_2H_5$.

Preferably Y is —S—.

$R^5$ is preferably $(CH_2)_nCOOH$ in which n is 4 to 10, more preferably 5.

The compounds of the invention may be prepared by methods analogous to those known in the art starting from the appropriate intermediate compounds. For example, the preparative routes disclosed in United States Patent Specification No. 2 493 748 may be employed.

The invention will now be illustrated by the following Examples.

## Example 1

3(5-carboxypentyl)-4-oxo-2-thioxo-thiazolidine (Intermediate compound)

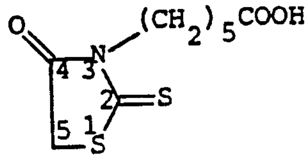

6-amino-hexanoic acid (131 g) was added to water (270 ml) and a 40% aqueous solution of sodium hydroxide added (140 ml). The mixture was cooled below 15°C whilst carbon disulphide (76.2 g) was slowly added with good stirring. Stirring was continued for approximately two hours and then some unreacted carbon disulphide was separated. A solution of chloroacetic acid (93.5 g) in water (170 ml) which had previously been made just alkaline (pH = 8) by the addition of sodium carbonate, was cooled to 5°C and then added. After mixing, the solution was left to stand overnight at room temperature before adding slowly to concentrated hydrochloric acid (S.G. 1.18) (275 ml), which was maintained by heating at 65 to 70°C. After the addition, the temperature was maintained at 90°C for half an hour.

Upon cooling to room temperature an initial oily deposit solidified and was filtered off, washed with water and dried.

The crude waxy solid was off-white in colour and exhibited a melting point of 70.5°C. A yield of 120 g was obtained.

## Example 2

3(5-carboxypentyl)-5(1-ethyl-1:4-dihydroquinolinylidene-4-ethylidene)-4-oxo-2-thioxo-thiazolidine

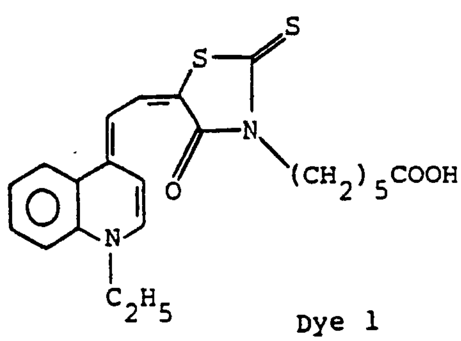

Dye 1

4-acetanilinovinyl-1-ethyl-quinolinium iodide (444 g) was added to 95% aqueous ethanol (2100 ml). 3(5-carboxypentyl)-4-oxo-2-thioxo-thiazolidine (274 g) was then added, followed by triethylamine (280 ml).

The mixture was heated under reflux for 15 minutes and then cooled to 55°C. Cold 2N aqueous hydrochloric acid (1000 ml) was added and after cooling to 40°C the mixture was filtered and the dye residue well pressed. The undried crude dye was placed in 95% aqueous methanol (4200 ml) containing 0.5 ml of 2N hydrochloric acid, heated to boiling for a few minutes and then filtered hot. The residue of dye was washed with more 95% aqueous methanol before drying in vacuuo at 55°C.

The yield of dark magenta needles was 222 g (52% theory). A solution in 90% aqueous methanol with a trace of triethylamine present exhibited $\lambda_{max}$ at 616 and 576 nm with $\varepsilon = 12.4 \times 10^4$ and $6.2 \times 10^4$ respectively.

| | N (%) | S (%) |
|---|---|---|
| Found | 6.54 | 14.94 |
| Calculated | 6.69 | 14.95 |

Example 3

3(5-carboxypentyl)-5(1-ethyl-1:2-dihydroquinolinylidene-2-ethylidene)-4-oxo-2-thioxo-thiazolidine

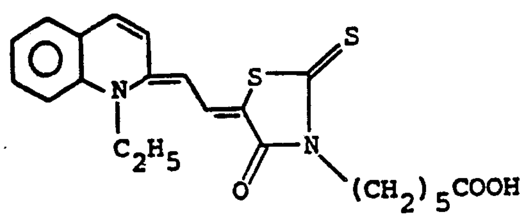

Dye 2

This dye was synthesised adopting the procedure of Example 2, with the exception that 2-acetanilinovinyl-3-ethyl-quinolinium iodide was substituted for the 4-acetanilinovinyl compound. The dye was obtained as a dark magenta powder exhibiting a melting point at 209°C in a yield of 25% theory.

A methanolic solution with triethylamine present exhibitied $\lambda_{max}$ at 566 and 534 nm with $\varepsilon = 7.9 \times 10^4$ and $7.45 \times 10^4$ respectively.

Example 4

Dye C—1 used in this Example is a trinuclear merocyanine dye disclosed in GB—A—1325312 presently used in some commercial embodiments of photothermographic emulsions and has the formula:

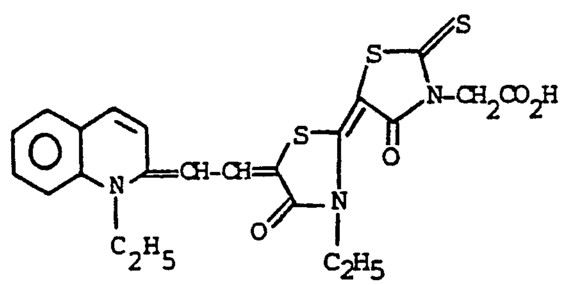

Dyes C—2 and C—3 used in this Example are compounds of the formula:

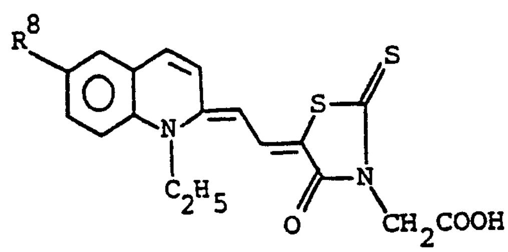

Dye C—2: $R^8 = H$
Dye C—3: $R^8 = C_2H_5O$

These comparative dyes were prepared in a similar manner to the methods described in Examples 2 and 3 starting from the appropriate quinoline derivative. In both cases the other reagent is 3-carboxymethyl-4-oxo-2-thioxo-thiazolidine, whose preparation is described in US 2,493,748.

To 700 g of a dispersion containing 12.5 parts of silver behenate, 6.5 parts of methyl isobutyl ketone, 21 parts of toluene, and 60 parts of butan-2-one maintained at 15°C with stirring was added the following sequence of materials at 15 minute intervals: 7 g of Butvar B—76 (poly(vinyl butyral) resins, Monsanto), 7 g of 1-methyl-1-pyrrolidone, 4 g of 0.5 molar mercuric bromide in ethanol, 20 g of 2 molal hydrobromic acid in ethanol, 70 g of Butvar B—76, 14 g of an antioxidant, and 7.6 g of phthalazinone. After 15 minutes stirring and digesting following the last addition, the emulsion was ready for dye sensitisation.

To separate 50 g aliquots of the emulsion were added 3.2 and 4.2 micromoles of each of the dyes under investigation. After 20 minutes digestion these aliquots were ready for coating. A convenient method for handling the dyes was as 0.3% to 1.0% solutions in 1-methyl-2-pyrrolidinone.

Using a knife coater with the orifice set 100 microns over a polyester web, two coatings were made from each aliquot and dried, each for 4 minutes as 90°C in a forced draft oven.

Next was applied a protective overcoat using the knife coater with the orifice set at 75 microns over the first trip and the coating dried as above. The overcoat solution contained 5 parts of a polyvinyl acetate-polyvinyl chloride copolymer (Union Carbide VYNS) and 95 parts butan-2-one.

Processing of several strips from each film sample was conducted at both 20 seconds and 60 seconds using either an inert fluid dip tank processor or heat surface processor maintained at 127°C. The superiority of the dyes of the invention in this formulation is seen in comparing the $D_{min}$ values obtained. Table 1 summarises these findings.

Table 1

| Film Sample | Dye | moles/ 50 g emulsion | $D_{min}$ Values 20 secs | | 60 seconds | | (replicate average) (60-20) | |
|---|---|---|---|---|---|---|---|---|
| | | | vis | W36 | vis | W36 | vis | W36 |
| 1,2 | C-1 | 3.2 | 0.11 | 0.17 | 0.20 | 0.28 | 0.09 | 0.11 |
| 3,4 | C-1 | 4.2 | 0.11 | 0.18 | 0.27 | 0.36 | 0.16 | 0.18 |
| 5,6 | C-2 | 3.2 | 0.10 | 0.17 | 0.16 | 0.24 | 0.07 | 0.08 |
| 7,8 | C-2 | 4.2 | 0.11 | 0.18 | 0.18 | 0.27 | 0.07 | 0.09 |
| 9,10 | 2 | 3.2 | 0.10 | 0.17 | 0.13 | 0.21 | 0.03 | 0.04 |
| 11,12 | 2 | 4.2 | 0.10 | 0.16 | 0.14 | 0.23 | 0.04 | 0.07 |

The improved response of the emulsions containing the dyes of the invention to the Eastman Kodak Wratten 36 filter, a measure of "duping $D_{min}$" encountered when one uses diazo or vesicular materials to make duplicates of original films, was particularly desirable. Of additional importance was the minimal effect on $D_{min}$ compared to the standard dye C—1. This is analogous to the effect seen when one overworks solutions during coating operations. The dye of the invention also exhibited improved results compared to the dye C—2 of similar structure.

Various other adjuvants may be added to the photothermographic emulsions of the present invention. For example, toners, accelerators, acutance dyes, sensitisers, stabilisers, surfactants, lubricants, coating aids, antifoggants, leuco dyes, chelating agents, and various other well known additives may be usefully incorporated.

A preferred silver halide emulsion was formed according to Example 1 of United States Patent Specification No. 4 161 408 using 7 mole percent silver bromochloride to 93 mole percent of silver behenate. The dyes under investigation were added to the emulsion immediately before coating. The samples were then oven dried at 90°F (32°C). The data are recorded in Table 2 with the concentration of the dye given as micromoles of dye per 50 grams of emulsion.

Table 2

| Dye | Conc. | $D_{min}$ | Relative Speed | | |
|---|---|---|---|---|---|
| | | | 600 nm | 620 nm | 640 nm |
| C-1 | 3 | 0.14 | 145 | 100 | 76 |
| C-1 | 3.6 | 0.21 | 137 | 106 | 114 |
| C-2 | 6.0 | 0.16 | 279 | 197 | 87 |
| C-2 | 7.2 | 0.17 | | | |
| C-3 | 7.2 | 0.20 | 134 | 186 | 100 |
| 2 | 4.8 | 0.17 | 335 | 305 | 172 |
| 2 | 9.6 | 0.19 | | | |

No readings for speed were taken at the higher dye concentrations for Dyes C—2 and 2. The dye concentrations used show that even as much as a two-fold increase in concentration of a dye according to the present invention can have less effect than a 20% variation in dye C—1 previously used to sensitise photothermographic emulsions. The dye of the invention also shows an improvement over dye C—2 which is of similar structure.

Example 5

Two emulsions were prepared in accordance with the procedure of Example 4, one containing Dye 1 and the other Dye C—1 in equimolar quantities. The emulsions were coated as in Example 4 and dried under a variety of temperature conditions. The 20-second and 60-second $D_{min}$ were measured after processing at 260°F (127°C) on a heated drum processor.

The following Table 3 reports the $D_{min}$ values for the emulsion containing Dye 1 for different drying temperatures.

Table 3

| Temperature (°F) | $D_{min}$ | |
|---|---|---|
| | 20-secs | 60-secs |
| 180 | 0.21 | 0.21 |
| 190 | 0.20 | 0.20 |
| 200 | 0.20 | 0.21 |
| 210 | 0.21 | 0.22 |
| 220 | 0.21 | 0.21 |
| 230 | 0.21 | 0.20 |
| 240 | 0.19 | 0.19 |

The results with Dye C—1 were similar at the lower drying temperatures but near 200°F (93°C) an increase of 0.1 to 0.15 in the 60-second $D_{min}$ per 10°F (5.6°C) temperature increase occurred. For drying temperatures of 220°F (104°C) and above, the 20-second $D_{min}$ began to increase and the 60-second $D_{min}$ became gross fog.

This Example demonstrates the good thermal stability of emulsions containing the dyes of the invention compared with an emulsion of the prior art.

Example 6

3(5-carboxypentyl)-5(1-methyl-1:4-dihydroquinolinyliene-4-ethylidene)-4-oxo-2-thioxo-thiazolidine

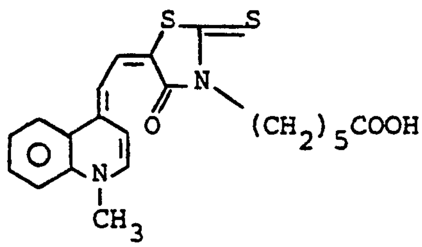

4-acetanilinovinyl-1-methyl-quinolinium iodide (8.88 g) was added to 95% aqueous ethanol (40 ml). 3(5-carboxypentyl)-4-oxo-2-thioxo-thiazolidine (3.75 g) was then added, followed by triethylamine (4.9 ml). The mixture was heated under reflux for 15 minutes and then cooled to 55°C. Cold 2N aqueous hyrochloric acid (17.5 ml) was added and after cooling to 40°C the mixture was filtered and the dye residue well pressed. The undried crude dye was placed in 95% aqueous methanol (100 ml) containing 0.1 ml of 2N hydrochloric acid, heated to boiling for a few minutes then filtered hot. The residue of dye was washed with more 95% aqueous methanol before drying in vacuo at 55°C. The yield of dark green crystals was 0.2 g.

A solution in 90% aqueous methanol with a trace of triethylamine present exhibited $\lambda_{max}$ at 615 nm with $\varepsilon = 12.3 \times 10^4$ and a supplementary peak at 576 nm. Elemental analysis:

| | C% | H% |
|---|---|---|
| Found | 60.64 | 5.44 |
| Calculated | 60.84 | 5.35 |

Example 7

3-ethyl-5[1(5-carboxypentyl)-1:4-dihydroquinolinylidene-4-ethylidene]-4-oxo-2-thioxo-thiazolidine

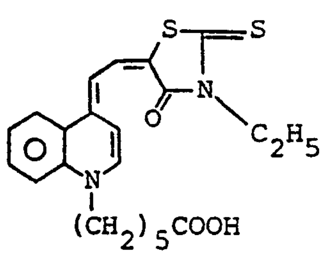

4-methyl-quinoline (7.15 g) and 6-bromohexanoic acid (9.76 g) were mixed and heated on a steam-bath for five hours. The material set to a gum upon cooling to room temperature. 5-acetanilinomethylene-3-ethyl-4-oxo-2-thioxo-thiazolidine (9.18 g), 95% aqueous ethanol (100 ml) and triethylamine (14 ml) were then added and the whole heated under reflux for 15 minutes. The resulting solution was cooled to 50°C and then 2N hydrochloric acid added (25 ml) followed by about 10 g of ice. Dye separated and the sludge-like mass was filtered.

The damp filter cake was washed by slurrying three times with 95% aqueous ethanol (50 ml) and finally extracted by boiling with 200 ml of the same solvent to leave dark green crystals (6.35 g). A 90% aqueous methanol solution containing a trace of triethylamine showed $\lambda_{max}$ at 616 and 575 nm with $\varepsilon = 10.8 \times 10^4$ and $6.3 \times 10^4$ respectively.

Elemental analysis:

| | C% | H% | S% |
|---|---|---|---|
| Found | 60.64 | 5.44 | 16.25 |
| Calculated | 60.84 | 5.35 | 15.47 |

**Claims**

1. A compound of the general formula:

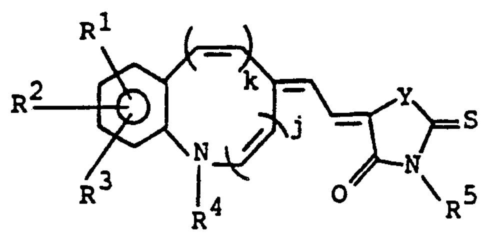

in which:

$R^1$, $R^2$ and $R^3$ independently represent a hydrogen atom or a substituent compatible with cyanine dyes,

$k + j = 1$,

$R^4$ represents a lower alkyl group containing 1 to 4 carbon atoms, $(CH_2)_nCOOX$, in which n is an integer from 1 to 10, and X is a cation,

Y = S or > N—$R^6$, and

$R^5$ and $R^6$ independently represent a lower alkyl group of 1 to 4 carbon atoms, cyclohexyl, phenyl, $(CH_2)_nCOOX$ in which n and X are as defined above,

with the proviso that at least one of $R^4$, $R^5$ and $R^6$ represents $(CH_2)_nCOOX$ in which n is at least 4.

2. A compound as claimed in Claim 1, characterised in that $R^1$, $R^2$ and $R^3$ are independently selected from hydrogen, halogen, alkyl of 1 to 4 carbon atoms, alkoxy of 1 to 4 carbon atoms, alkenyl of 2 to 4 carbon atoms, alkaryl, aralkyl, phenyl, $—(CH_2)_mCOOH$ in which m is 0, 1, 2 or 3, $—NO_2$, $—NH_2$ or $NHCOCH_3$.

3. A compound as claimed in Claim 1 or Claim 2, characterised in that at least two of $R^1$ to $R^3$ represent hydrogen.

4. A compound as claimed in Claim 3 characterised in that each of $R^1$ to $R^3$ represent hydrogen.

5. A compound as claimed in any preceding claim, characterised in that $R^4$ is $C_2H_5$.

6. A compound as claimed in any preceding claim characterised in that Y is —S—.

7. A compound as claimed in any preceding claim, characterised in the $R^5$ represents $(CH_2)_nCOOX$ in which n is 4 to 10.

8. A compound as claimed in Claim 7, characterised in that n is 5.

9. 3(5-carboxypentyl)-5(1-ethyl-1:4-dihydroquinolinylidene-4-ethylidene)-4-oxo-2-thioxo-thiazolidine.

10. 3(5-carboxypentyl)-5(1-ethyl-1:2-dihydroquinolinylidene-2-ethylidene)-4-oxo-2-thioxo-thiazolidine.

11. 3(5-carboxypentyl)-5(1-methyl-1:4-dihydroquinolinylidene-4-ethylidene)-4-oxo-2-thioxo-thiazolidine.

12. 3-ethyl-5[1(5-carboxypentyl)-1:4-dihydroquinolinylidene-4-ethylidene]-4-oxo-2-thioxo-thiazolidine.

**Patentansprüche**

1. Eine Verbindung der allgemeinen Formel

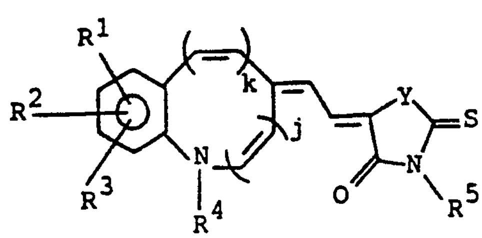

in der

$R^1$, $R^2$ und $R^3$ unabhängig voneinander ein Wasserstoffatom oder einen mit Cyanin-Farbstoffen verträglichen Substituenten bedeuten,

k + j = 1,

$R^4$ einen Niederalkylrest mit 1 bis 4 Kohlenstoffatomen, $(CH_2)_nCOOX$, wobei n eine ganze Zahl von 1 bis 10 und X ein Kation ist, darstellt,

Y = S oder > N—$R^6$, und

$R^5$ und $R^6$ unabhängig voneinander einen Niederalkylrest mit 1 bis 4 Kohlenstoffatomen, Cyclohexyl, Phenyl, $(CH_2)_nCOOX$, wobei n und X wie vorstehend definiert sind, darstellen,

mit der Maßgabe, daß mindestens einer der Reste $R^4$, $R^5$ und $R^6$ $(CH_2)_nCOOX$ bedeutet wobei n mindestens 4 ist.

2. Verbindung nach Anspruch 1, dadurch gekennzeichnet, daß $R^1$, $R^2$ und $R^3$ unabhängig voneinander Wasserstoff, Halogen, Alkyl mit 1 bis 4 Kohlenstoffatomen, Alkoxy mit 1 bis 4 Kohlenstoffatomen, Alkenyl mit 2 bis 4 Kohlenstoffatomen, Alkaryl, Aralkyl, Phenyl, $(CH_2)_mCOOH$, wobei m 0, 1, 2 oder 3 ist, —$NO_2$, —$NH_2$ oder —$NHCOCH_3$ bedeuten.

3. Verbindung nach Anspruch 1 oder Anspruch 2, dadurch gekennzeichnet, daß mindestens zwei der Reste $R^1$ bis $R^3$ Wasserstoff bedeuten.

4. Verbindung nach Anspruch 3, dadurch gekennzeichnet, daß jeder der Reste $R^1$ bis $R^3$ Wasserstoff bedeutet.

5. Verbindung nach einem der vorangehenden Ansprüche, dadurch gekennzeichnet, daß $R^4$ —$C_2H_5$ ist.

6. Verbindung nach einem der vorangehenden Ansprüche, dadurch gekennzeichnet, daß Y —S— ist.

7. Verbindung nach einem der vorangehenden Ansprüche, dadurch gekennzeichnet, daß $R^5$ $(CH_2)_nCOOX$ bedeutet, wobei n 4 bis 10 ist.

8. Verbindung nach Anspruch 7, dadurch gekennzeichnet, daß n 5 ist.

9. 3-(5-Carboxypentyl)-5-(1-äthyl-1:4-dihydrochinolinyliden-4-äthyliden)-4-oxo-2-thioxo-thiazolidin.

10. 3-(5-Carboxypentyl)-5-(1-äthyl-1:2-dihydrochinolinyliden-2-äthyliden)-4-oxo-2-thioxo-thiazolidin.

11. 3-(5-Carboxypentyl)-5-(1-methyl-1:4-dihydrochinolinyliden-4-äthyliden)-4-oxo-2-thioxo-thiazolidin.

12. 3-Athyl-5-[1-(5-carboxypentyl)-1:4-dihydrochinolinyliden-4-äthyliden]-4-oxo-2-thioxo-thiazolidin.

**Revendications**

1. Composé de formule générale:

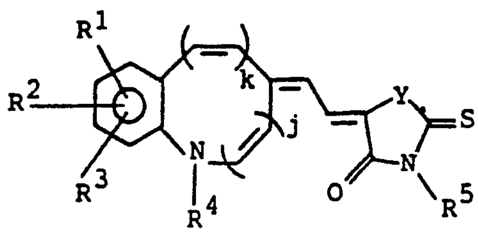

dans laquelle:

$R^1$, $R^2$ et $R^3$ indépendamment les uns des autres, représentent chacun un atome d'hydrogène ou un substituant compatible avec les colorants du type cyanine,

k + j = 1,

$R^4$ représente un groupe alkyle inférieur contenant de 1 à 4 atomes de carbone, ou un groupe $(CH_2)_nCOOX$ où n est un nombre entier ayant pour valeur 1 à 10 et X est un cation,

Y est S ou N—$R^6$, et

$R^5$ et $R^6$ indépendamment l'une de l'autre représentent chacun un groupe alkyle inférieur en $C_1$—$C_4$, cyclohexyle, phényle ou $(CH_2)_nCOOX$ où n et X sont définis comme ci-dessus, sous réserve qu'au moins un des $R^4$, $R^5$ et $R^6$ représente le groupe $(CH_2)_nCOOX$ où n vaut au moins 4.

2. Composé selon la revendication 1, caractérisé en ce que $R^1$, $R^2$ et $R^3$ sont indépendamment choisis parmi l'atome d'hydrogène, les atomes d'halogène, les groupes alkyle en $C_1$—$C_4$, les groupes alcoxy en $C_1$—$C_4$, les groupes alcényle en $C_2$—$C_4$, les groupes alkaryle, les groupes aralkyle, le groupe phényle, les groupes —$(CH_2)_mCOOH$ où m vaut 0, 1, 2 ou 3, les groupes —$NO_2$, —$NH_2$ ou —$NHCOCH_3$.

3. Composé selon la revendication 1 ou la revendication 2, caractérisé en ce qu'au moins deux des groupes $R^1$ à $R^3$ représentent chacun l'atome d'hydrogène.

4. Composé selon la revendication 3, caractérisé en ce que chacun des groupes $R^1$ à $R^3$ représente l'atome d'hydrogène.

5. Composé selon l'une quelconque des revendications précédentes, caractérisé en ce que $R^4$ est $C_2H_5$.

6. Composé selon l'une quelconque des revendications précédentes, caractérisé en ce que Y est —S—.

7. Composé selon l'une quelconque des revendications précédentes, caractérisé en ce que $R^5$ représente $(CH_2)_nCOOX$ où n a pour valeur 4 à 10.

8. Composé selon la revendication 7, caractérisé en ce que n a pour valeur 5.

9. 3-(5-carboxypentyl)-5-(1-éthyl-1:4-dihydroquinolinylidène-4-éthylidène)-4-oxo-2-thioxo-thiazolidine.

10. 3 - (5 - carboxypentyl) - 5 - (1 - éthyl - 1:2 - dihydroquinolinylidène - 2 - éthylidène) - 4 - oxo - 2 - thioxo - thiazolidine.

11. 3 - (5 - carboxypentyl) - 5 - (1 - méthyl - 1:4 - dihydroquinolinylidène - 4 - éthylidène) - 4 - oxo - 2 - thioxo - thiazolidine.

12. 3 - éthyl - 5 - [1 - (5 - carboxypentyl) - 1:4 - dihydroquinolinylidène - 4 - éthylidène] - 4 - oxo - 2 - thioxo - thiazolidine.